# EUROPEAN PATENT APPLICATION

(11) **EP 3 805 374 A1**
(43) Date of publication of application: **14.04.2021**
(21) Application number: 19810230.3
(22) Date of filing: 31.05.2019
(51) Int. Cl.: C12N 5/0797, C12N 5/079

(54) **DIFFERENTIATION MEDIUM AND METHOD FOR PREPARING OLIGODENDROCYTE PRECURSOR**

(30) Priority: 01.06.2018 CN 201810553985
(71) Applicant: Help Stem Cell Innovations Co., Ltd., Nanjing, Jiangsu 211112 (CN)
(72) Inventor: TSUI, Yat Ping, Nanjing, Jiangsu 211112 (CN); WANG, Jiaxian, Nanjing, Jiangsu 211112 (CN); CHAN, Ying Shing, Nanjing, Jiangsu 211112 (CN); SHUM, Kwok Yan Daisy, Nanjing, Jiangsu 211112 (CN); WU, Lap Kei Kenneth, Nanjing, Jiangsu 211112 (CN); LAM, Guy, Nanjing, Jiangsu 211112 (CN)
(74) Representative: Epping - Hermann - Fischer
(86) International application number: PCT/CN2019/089625
(87) International publication number: WO 2019/228518

(57) **Abstract**

Disclosed are a medium for differentiating neural stem cells into oligodendrocyte precursors and a method for preparing oligodendrocyte precursors by using the medium. The medium does not contain exogenous factors, and can avoid the contamination of exogenous factors and differentiate oligodendrocyte precursors.

## Description

### FIELD

The present invention relates to the technical field of cell differentiation, in particular to a medium and a method of preparing oligodendrocyte precursor cells.

### BACKGROUND

The myelin sheath is an important constitution structure in the nervous system. Demyelinating diseases can cause dysfunctions of cognition, memory and motor, which seriously affect the life quality of patients.

The myelin sheath is formed by oligodendrocytes (OLs) winding around the axons of neurons in the vertebrate central nervous system (CNS). In the early stages of neural development, neural precursor cells first produce neurons and then oligodendrocyte precursor cells. Oligodendrocyte precursor cells migrate and proliferate to other parts of the nervous system, and finally differentiate into mature oligodendrocytes and form myelin sheath.

At present, both *in vitro* and *in vivo* experiments have demonstrated that oligodendrocyte precursor cells have the ability to proliferate, migrate and differentiate to form myelin sheath, so the transplantation of oligodendrocyte precursor cells is an effective way to treat myelin sheath injury diseases. Recent studies have shown that the hotspot in the application of oligodendrocyte precursor cells transplantation is to repair the spinal cord injury related to myelin sheath injury.

With the deepening research on pluripotent stem cells, the problem of oligodendrocyte precursor cells source has been solved. At present, the differentiation of pluripotent stem cells into oligodendrocyte precursor cells mainly refers to and mimics the natural development of oligodendrocytes in the body. First, pluripotent stem cells are induced to differentiate into neuroepithelial cells, and then further differentiated into oligodendrocyte precursor cells. However, the current methods for inducing pluripotent stem cells to oligodendrocyte precursor cells are complicated and time-consuming. The entire culture process takes about 4 months, or even 6 months, which significantly limits the application of oligodendrocyte precursor cells in therapy or constructing disease models. In current culture methods of differentiating neuroepithelial cells into oligodendrocyte precursor cells, the media are usually added foreign substances such as fetal bovine serum, non-human albumin or non-human growth factors, which increases the risk of oligodendrocyte precursor cells in clinical application. At present, many researchers are dedicated to develop a medium and culturing method that can shorten the differentiation time and improve the differentiation efficiency.

### SUMMARY

In order to improve the efficiency of preparing oligodendrocyte precursor cells without exogenous materials, in one aspect, the present invention provides in the examples a medium for differentiating neural stem cells into oligodendrocyte precursor cells, which comprises a basal medium mixed from Advanced DMEM/F12 and Neurobasal medium, as well as 0.5~2.5% GlutaMAX, 1~5% B27, 0.5~2.5%N2, 2~25 ng/ml bFGF, 2~25 ng/ml PDGF-AA, 25~250 ng/ml β-HRG1, 1-20 µM 2-mercaptoethanol, and 1% penicillin-streptomycin (P/S) at a final concentration.

The medium for differentiating neural stem cells into oligodendrocyte precursor cells provided by the present invention shortens the time of differentiation from neural stem cells to oligodendrocyte precursor cells, and improves the differentiation efficiency of neural stem cells into oligodendrocyte precursor cells. Wherein, 2-mercaptoethanol added in the medium can not only improve the antioxidant capacity of cells and enhance cell viability, but also work together with PDGF-AA and β-HRG1 in the medium to significantly improve the differentiation efficiency of oligodendrocyte precursor cells.

In order to further improve the differentiation efficiency of oligodendrocyte precursor cells and the purity of the final products, preferably, the medium, which is used to differentiate neural stem cells into oligodendrocyte precursor cells, comprises a basal medium mixed from Advanced DMEM/F12 and Neurobasal medium at a volume ratio of 1:1, as well as 1% GlutaMAX, 2% B27, 1% N2, 10 ng/ml bFGF, 10 ng/ml PDGF-AA, 100 ng/ml β-HRG1, 10 µM 2-mercaptoethanol, and 1% P/S at a final concentration.

Further preferably, the medium comprises a basal medium mixed from Advanced DMEM/F12 and Neurobasal medium, as well as 0.5~2.5%GlutaMAX, 1~5% B27, 0.5~2.5%N2, 2~25 ng/ml bFGF, 2~25 ng/ml PDGF-AA, IGF-1, Wnt3A, 25-250 ng/ml β-HRG1, 1-20 µM 2-mercaptoethanol, and 1% P/S at a final concentration.

Adding IGF-1 and Wnt3A to the medium can further improve cell activity, increase the output of oligodendrocyte precursor cells, promote the differentiation of neural stem cells into glial cells, and further improve the differentiation efficiency, wherein the concentration of IGF-1 and Wnt3A in the medium is preferably 2-25 ng/ml and 1-10 ng/ml, respectively.

In order to further improve the differentiation efficiency of oligodendrocyte precursor cells and increase the production, the medium, which is used to differentiate neural stem cells into oligodendrocyte precursor cells, comprises a basal medium mixed from Advanced DMEM/F12 and Neurobasal medium at a volume ratio of 1:1, as well as 1% GlutaMAX, 2% B27, 1% N2, 10 ng/ml bFGF, 10 ng/ml PDGF-AA, 10 ng/ml IGF-1, 5 ng/ml Wnt3A, 100 ng/ml β-HRG1, 10 µM 2-mercaptoethanol and 1% P/S at a final concentration.

In another aspect, the present invention provides a method of preparing oligodendrocyte precursor cells, which comprises the steps of:

preparing a medium comprising a basal medium mixed from Advanced DMEM/F12 and Neurobasal medium, as well as 0.5~2.5%GlutaMAX, 1~5% B27, 0.5~2.5%N2, 2~25 ng/ml bFGF, 2~25 ng/ml PDGF-AA, 25-250 ng/ml β-HRG1, 1-20 µM 2-mercaptoethanol, and 1% P/S at a final concentration;
coating a cell culturing container with 2.5-25 µg/ml Poly-L-Omithine (PLO) and 2.5-25 µg/ml laminin, adding the medium of the above step, seeding neural stem cells into the container at a density of 1 x 10⁴ to 5 × 10⁴ cells/cm²; and
under the conditions of ensuring that the cells have sufficient growth factors and nutrients, replacing the medium regularly until oligodendrocyte precursor cells appear in the medium; or replacing the medium every 48h.

In the differentiation process of oligodendrocyte precursor cells, the interaction between cells affects the differentiation efficiency. Seeding cells at a density of 1 × 10⁴ to 5 × 10⁴ cells/cm² can increase the differentiation efficiency on the basis of ensuring the number of oligodendrocyte precursor cells. If the seeding density is higher than 5 × 10⁴ cells/cm² or lower than 1 x 10⁴ cells/cm², it takes longer differentiation time to obtain the same number of oligodendrocyte precursor cells.

In order to make the differentiated oligodendrocyte precursor cells close to or meet the clinical application standards, the laminin used to coat the cell culturing container is preferably human laminin.

More preferably, in the method of preparing oligodendrocyte precursor cells, neural stem cells are seeded into the cell culturing container at a density of 4 x 10⁴ cells/cm². Under this condition, about 5 days from the date of seeding, oligodendrocyte precursor cells can be observed in the culture; on the 8^{th} day, oligodendrocyte precursor cells with a purity higher than 90% can be obtained according to the means known by those skilled in the art. The obtained cells can be further used in clinical research or used as active ingredient to treat diseases related to myelin sheath damage.

Preferably, in the method of preparing oligodendrocyte precursor cells, the cell culturing container is a 6-well plate. Under the premise of ensuring cell density, other cell culture plates or culture dishes are not excluded, such as 12-well plates, 24-well plates, or cell culturing dishes according to the actual amount of oligodendrocytes needed to be differentiated.

The neural stem cells used in the method of preparing oligodendrocyte precursor cells in the examples of the present invention may be neural stem cells prepared according to various methods well known to those skilled in the art. Preferably, the neural stem cells are derived from bone marrow mesenchymal stem cells. The oligodendrocyte precursor cells prepared from bone marrow mesenchymal stem cell according to the method provided by the present invention are capable of being applied to the treatment of myelin sheath damage across species without obvious rejection or other researches.

Another object of the present invention is to provide a method that can stably control the process from bone marrow mesenchymal stem cells to oligodendrocyte precursor cells and improve the quality of oligodendrocyte precursor cells.

Preferably, in the method of preparing oligodendrocyte precursor cells, the preparation of neural stem cells comprises the following steps:

preparing a medium for neural stem cells, wherein the medium is Advanced DMEM/F12 medium containing 0.5%~2.5% GlutaMAX, 1%~5% B27, 10~60 ng/ml bFGF, 10~60 ng/ml EGF, 5~40 ng/ml IGF-1 and 1 % P/S at a final concentration;
culturing bone marrow mesenchymal stem cells by non-adherent culture using the medium for neural stem cells, and seeding the cells into a non-adherent cell culturing container at a density of 7,500 to 20,000 cells/cm²; and
replacing the medium for neural stem cells regularly according to the growth status of the cells, wherein the neural stem cells appear in a form of neurosphere in the medium.

More preferably, in the method of preparing oligodendrocyte precursor cells, preparing a medium for neural stem cells, wherein the medium is Advanced DMEM/F12 medium containing 1% GlutaMAX, 2% B27, 40 ng/ml bFGF, 40 ng/ml EGF, 20 ng/ml IGF-1 and 1 % P/S at a final concentration; and culturing bone marrow mesenchymal stem cells by non-adherent culture using the medium for neural stem cells, and seeding the cells into a non-adherent cell culturing container at a density of 10,000 cells/cm². Wherein, higher concentrations of bFGF and EGF combined with IGF-1 can effectively increase the numbers of neurospheres.

The neurospheres should be cultured in a non-adherent manner in the culturing container, which can inhibit the autonomous differentiation of neurospheres and help control the differentiation process. When the non-adherent culturing method is used, the neurospheres are collected without enzymatic digesting the cells, and the culture can be directly centrifuged, which reduces the damage to the cells in the digestion step. Therefore, culturing bone marrow mesenchymal stem cells in a non-adherent manner can increase the yield of oligodendrocyte precursor cells prepared from bone marrow mesenchymal cells.

Preferably, in the method of preparing oligodendrocyte precursor cells, the neural stem cells are present in the form of neurospheres in the medium for neural stem cells, and the neurospheres can be separated from the medium under the condition of centrifugation at 100 g to 300 g for about 5 min.

Preferably, in the method of preparing oligodendrocyte precursor cells, the bone marrow mesenchymal stem cells are prepared from bone marrow by the following steps:
culturing bone marrow in Stem Pro Medium, removing the medium and non-adherent cells 48h later, continuing culturing by adding fresh medium; and
after colonies of bone marrow mesenchymal stem cells appear in the medium, seeding the cells at a density of 40,000 cells/cm² into Stem Pro Medium for passage culturing, until the amount of CD45-positive cells in the culture is less than 1%.

Bone marrow contains many different cells other than bone marrow mesenchymal stem cells. After 48 hours of adherent culture of bone marrow, removing the medium can greatly reduce non-adherent other cells and improve the purity of bone marrow mesenchymal stem cells.

Through experiments, the inventors found that hematopoietic stem cells in the bone marrow affect the efficiency of the differentiation of bone marrow mesenchymal stem cells to produce neural stem cells. After 48 hours of bone marrow culturing, subculture of adherent cells can greatly reduce the hematopoietic stem cells in the bone marrow. Under normal conditions, passage 3 to 4 times can make the amount of CD45-positive cells less than 1%.

In addition, passaging at a seeding density of 40,000 cells/cm² helps maintain the property of bone marrow mesenchymal stem cells, inhibits the random differentiation of bone marrow mesenchymal stem cells, thereby increasing the yield of oligodendrocyte precursor cells prepared from bone marrow.

Further preferably, in the method of preparing oligodendrocyte precursor cells, in the bone marrow mesenchymal stem cells used for preparing the neural stem cells, the amount of STRO-1, CD90 and CD73-positive cells is greater than 90%, the amount of Nestin-positive cells is greater than 5%, and the amount of CD45-positive cells is less than 1%.

The amount of cell marker positive expression in the medium can be controlled by the number of passage of bone marrow mesenchymal rod cells. When the amount of expression of the CD45-positive cells in medium is less than 1%, the influence of hematopoietic stem cells on the production of neural stem cells from bone marrow mesenchymal stem cells is negligible.

The present invention provides a medium with the ability of efficiently differentiating oligodendrocyte precursor cells, and a method of preparing oligodendrocyte precursor cells using the medium, including a method of preparing oligodendrocyte precursor cells from bone marrow. In the medium and the method of preparing oligodendrocyte precursor cells provided by the present invention, no exogenous factors are used, thus avoiding contamination by exogenous factors and providing possibility for clinical studies. In addition, the preparation method provided by the invention also has the beneficial effects of high yield of preparing oligodendrocyte precursor cells, high differentiation efficiency and controllable differentiation process.

### BRIEF DESCRIPTION OF DRAWINGS

Figure 1 shows the flow cytometry detection of bone marrow mesenchymal stem cell markers CD90, CD73, STRO-1, CD45 and Nestin in Example 1.
Figure 2 is a bar graph showing the proportions of Nestin-positive cells and GFAP-positive cells in the total number of cells in neurospheres, taking the positive expression amount of Nestin and GFAP in bone marrow mesenchymal stem cells before seeding into neural stem cell medium of Example 2 as a control.

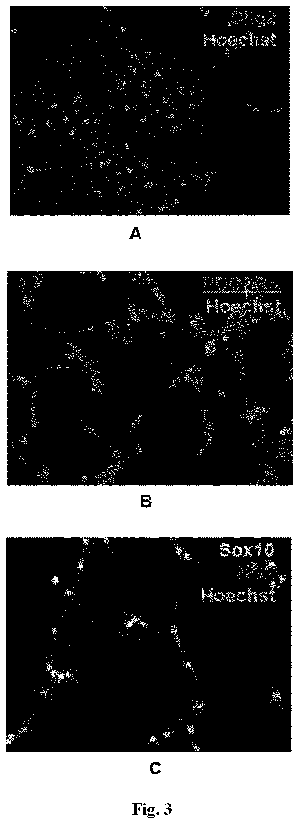

Figure 3 shows images of immunofluorescence staining of Olig2, PDGFRα, NG2 and Sox10 in the oligodendrocyte precursor cells prepared according to Example 4.
Figure 4 shows the flow cytometry detection of oligodendrocyte precursor cell markers Olig2, PDGFRα, NG2 and Sox10 in Example 4.
Figure 5 shows the expression of myelin basic protein (MBP) in shiverer model mice 12 weeks after injection of oligodendrocyte precursor cells (panel B), taking CIB (Cell Injection Buffer) without oligodendrocyte precursor cells as a control (panel A).
Figure 6 shows the results of life extension of shiverer model mice injected with human oligodendrocyte precursor cells and rat oligodendrocyte precursor cells, respectively.

### DETAILED DESCRIPTION

The present invention will be further described below in conjunction with the drawings and specific examples. The experimental methods in the examples, unless otherwise specified, are all conventional methods, and the instruments and reagents used in the experiments are all commercially available.

For the main components of the medium used in the examples of the present invention, see the following source information:
CTS Stem Pro Medium: Thermo Fisher, Cat. No.: A1033201
Advanced DMEM/F12: Thermo Fisher, Cat. No.: 12634028
Neurobasal medium: Thermo Fisher, Cat. No.: 21103049

In the examples of the present invention, clinical standard growth factors are used, such as CTS N2 (Thermo Fisher, Cat. No.: 1370701), CTS B27 (Thermo Fisher, Cat. No.: 1486701) GMP bFGF (Peprotech, Cat. No.: GMP100) -18B) and GMP PDGF-AA (Peprotech, Cat. No.: GMP100-13A). It is not excluded that non-clinical standard chemical reagents are used in the examples of the present invention to prepare oligodendrocyte precursor cells.

### Example 1: Pretreatment of Bone Marrow Mesenchymal Stem Cells (BMSC)

1. Materials sources: Bone marrow sources included, but not limited to, 30-day old Sprague-Dawley rats, or human adults aged 18-40.
2. 1 ml bone marrow from Sprague-Dawley rat or human adult was diluted with 9 ml of CTS Stem Pro Medium, and the diluted bone marrow was added to a 10 cm cell culture dish after modification treatment suitable for adherent cell culture. The day when bone marrow was subjected to adherent culture was defined as the first day (D0) of the differentiation of oligodendrocyte precursor cells.
3. After 48 h, the medium in the cell culture dish was removed, the non-adherent cells were washed away with DPBS, and 10 ml CTS Stem Pro Medium was added. Thereafter, the CTS Stem Pro Medium was replaced every 72 h to continue the culturing.
4. At D6 to D7 of the culturing, bone marrow mesenchymal stem cell (BMSC) colonies could be observed in the dish. At D8 to D10 of the culturing, the medium was sucked up and removed, and the non-adherent cells in the dish were washed with 3 ml of DPBS; the DPBS was discarded, and 1 ml of CTS Tryple Select was added to the dish; the dish was incubated at 37°C for 5 min, the digested cell solution was collected by centrifuging at 200 g for 5 min at room temperature to obtain pellet, and the pellet was resuspended in CTS Stem Pro Medium.
5. The above cells were seeded into a 6 cm or 10 cm culture dish at a density of 4x10⁴ cells/cm².
6. When the BMSCs in the dish reached 80%~90% confluence, the cells were subcultured, and the passage number of BMSCs could not exceed 10.
7. Identification of BMSC

### Detection of markers by flow cytometry

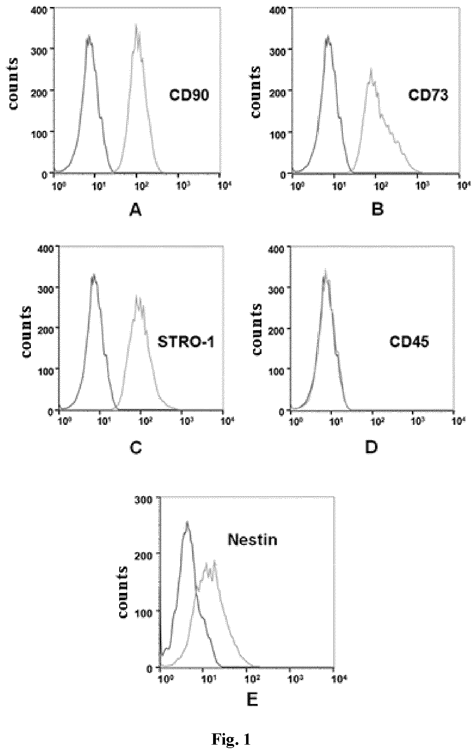

When BMSCs were passaged to P3, the cells were collected and digested routinely. After the cells were resuspended in DPBS, 4% PFA was added to fix the cells for 10 min. The cells were centrifuged and washed with DPBS for 2 to 3 times to remove PFA. The cells were incubated with blocking buffer containing primary antibodies CD90, CD73, CD45, Nestin and STRO-1 for 2 h. After centrifugation, the cells were incubated with blocking buffer containing fluorescent dye (Alexa488) labeled secondary antibody for 30 min. After centrifugation, the cells were resuspended in DPBS, and the cell surface antigens were detected by flow cytometry. In the negative control, a non-specific antibody from the same species with the same Ig type and label as the primary antibody was used as the isotype control. The flow cytometry results of Figure 1 show that the BMSCs prepared in the examples of the present invention express stably CD73, CD90 and STRO-1, and the BMSCs barely contain hematopoietic stem cells.

The blocking buffer was DPBS containing 2% BSA and 0.1% Triton X-100.

The following three groups of markers were detected using flow cytometry:
group A: BMSC markers: STRO-1, CD90 and CD73
group B: Neural stem cell marker: Nestin
group C: Hematopoietic stem cell marker: CD45

When more than 90% of cells in the dish were positive for group A markers, more than 5% of cells in the dish were positive for group B marker, and less than 1% of cells in the dish were positive for group C marker, the cells in the dish may be used for further operations.

### Example 2: Differentiation of BMSCs into Neural Stem Cells

### 1. Preparation of medium for neural stem cells

Advanced DMEM/F12 medium containing 1% CTS GlutaMAX, 2% CTS B27, 40 ng/ml GMP bFGF, 40 ng/ml GMP EGF, 20 ng/ml GMP IGF-1 and 1% P/S at a final concentration was prepared.
2. When BMSCs were passaged between the 3^{rd} and 10^{th} passages (preferably D15), the cells were digested with CTS Tryple Select, centrifuged, and resuspended in the medium for neural stem cells. The cells were seeded at a density of 1 x 10⁴cells/cm² to a Ultra Low® non-adherent 6-well plate.
3. The medium for neural stem cells was replaced every 48 hours. As the culturing time increased, neural stem cells formed neurospheres and suspended in the medium. When non-adherent culturing reached the 5^{th} to 7^{th} day, the medium was centrifuged at 200 g for 5 min to obtain neural stem cells in the form of neurospheres.

### Example 3: Identification of Neural Stem Cells

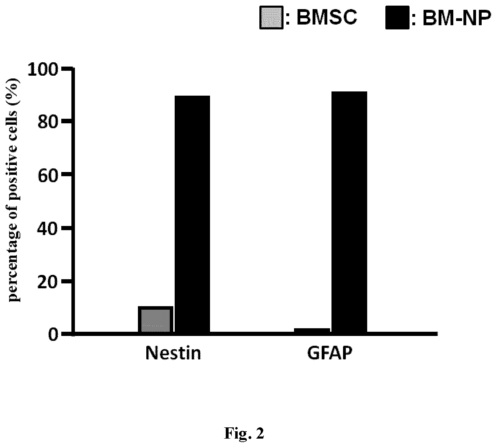

Using bone marrow mesenchymal stem cells as a control, the neurospheres on the 5^{th} day of the non-adherent culturing described in Example 2 were broken up, and the content of Nestin-positive and GFAP-positive cells in the neurospheres were detected by immunofluorescence method. It could be seen from Figure 2 that the amount of Nestin-positive and GFAP-positive cells in Example 2 exceeded 85%.

Experiments demonstrated that when the amount of Nestin-positive and GFAP-positive cells in the neurosphere is higher than 75%, the purity and differentiation efficiency of oligodendrocyte precursor cells obtained by further differentiation of the neurosphere are higher.

### Example 4: Differentiation of Neural Stem Cells into Oligodendrocyte Precursor Cells

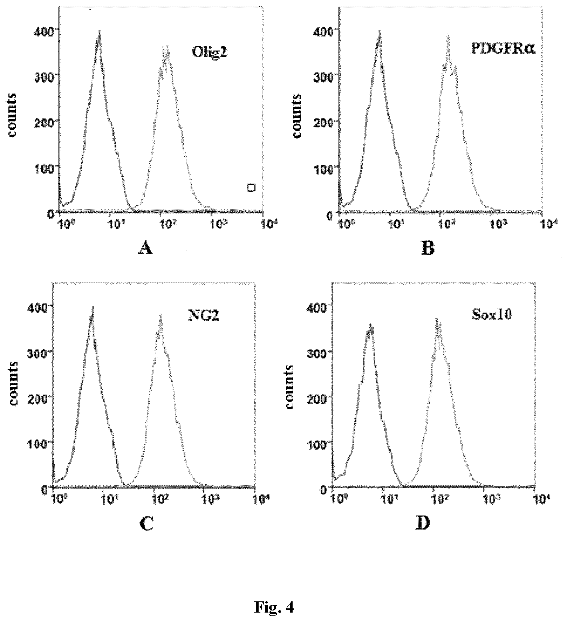

1. Preparation of differentiation medium for oligodendrocyte precursor cells
   The following 10 groups of differentiation medium for oligodendrocyte precursor cells were respectively prepared for the differentiation of the neural stem cells obtained in Example 2.
   (1) Group 1: comprising a basic medium made by mixing evenly Advanced DMEM/F12 and CTS Neurobasal medium at a volume ratio of 1:1, as well as 1% CTS GlutaMAX, 2% CTS B27, 1% CTS N2 10ng/ml GMP bFGF, 10 ng/ml GMP PDGF-AA, 100ng/ml GMP β-HRG1, 10µM 2-mercaptoethanol and 1% P/S at a final concentration, respectively.
   (2) Group 2: comprising a basic medium made by mixing evenly Advanced DMEM/F12 and CTS Neurobasal medium at a volume ratio of 9:2, as well as 0.5% CTS GlutaMAX, 1% B27, 0.5% N2, 2 ng/ml bFGF, 2 ng/ml PDGF-AA, 25ng/ml β-HRG1, 1 µM 2-mercaptoethanol, and 1% P/S at a final concentration, respectively.
   (3) Group 3: comprising a basic medium made by mixing evenly Advanced DMEM/F12 and CTS Neurobasal medium at a volume ratio of 1.5:7.5, as well as 2.5% CTS GlutaMAX, 5% B27, 2.5% N2, 25 ng/ml bFGF, 25 ng/ml PDGF-AA, 250ng/ml β-HRG1, 20 µM 2-mercaptoethanol, and 1% P/S at a final concentration, respectively.
   (4) Group 4: comprising a basic medium made by mixing evenly Advanced DMEM/F12 and CTS Neurobasal medium at a volume ratio of 1:1, as well as 1% CTS GlutaMAX, 2% CTS B27, 1% CTS N2, 10ng/ml GMP bFGF, 10 ng/ml GMP PDGF-AA, 2 ng/ml IGF-1, 25 ng/ml Wnt3A, 100ng/ml GMP β -HRG1, 10µM 2-mercaptoethanol and 1% P/S at a final concentration.
   (5) Group 5: comprising a basic medium made by mixing evenly Advanced DMEM/F12 and CTS Neurobasal medium at a volume ratio of 1:1, as well as 1% CTS GlutaMAX, 2% CTS B27, 1% CTS N2, 10ng/ml GMP bFGF, 10 ng/ml GMP PDGF-AA, 25 ng/ml IGF-1, 2 ng/ml Wnt3A 100ng/ml GMP β- HRG1, 10µM 2-mercaptoethanol and 1% P/S at a final concentration.
   (6) Group 6: comprising a basic medium made by mixing evenly Advanced DMEM/F12 and CTS Neurobasal medium at a volume ratio of 1:1, as well as 1% CTS GlutaMAX, 2% CTS B27, 1% CTS N2, 10ng/ml GMP bFGF, 10 ng/ml GMP PDGF-AA, 15 ng/ml IGF-1, 10 ng/ml Wnt3A, 100ng/ml GMP β -HRG1, 10µM 2-mercaptoethanol and 1% P/S.
   (7) Group 7: comprising a basic medium made by mixing evenly Advanced DMEM/F12 and CTS Neurobasal medium at a volume ratio of 1:1, as well as 1% CTS GlutaMAX, 2% CTS B27, 1% CTS N2, 10ng/ml GMP bFGF, 10 ng/ml GMP PDGF-AA, 100 ng/ml GMP β-HRG1 and 1% P/S at a final concentration.
   (8) Group 8: comprising a basic medium made by mixing evenly Advanced DMEM/F12 and CTS Neurobasal medium at a volume ratio of 1:1, as well as 1% CTS GlutaMAX, 2% CTS B27, 1% CTS N2, 10ng/ml GMP bFGF, 100ng/ml GMP β-HRG1, 10µM 2-mercaptoethanol and 1% P/S at a final concentration.
   (9) Group 9: comprising a basic medium made by mixing evenly Advanced DMEM/F12 and CTS Neurobasal medium at a volume ratio of 1:1, as well as 1% CTS GlutaMAX, 2% CTS B27, 1% CTS N2, 10ng/ml GMP bFGF, 10 ng/ml GMP PDGF-AA, 10µM 2-mercaptoethanol, and 1% P/S at a final concentration.
   (10) Group 10: comprising a basic medium made by mixing evenly Advanced DMEM/F12 and CTS Neurobasal medium at a volume ratio of 1:1, as well as 1% CTS GlutaMAX, 2% CTS B27, 1% CTS N2, 10ng/ml GMP bFGF, 10 ng/ml GMP PDGF-AA, 10 ng/ml NT-3 (Neurotrophic factor 3), 10µM 2-mercaptoethanol and 1% P/S at a final concentration.
2. The 6-well plate was coated with Poly-L-Ornithine (PLO) and human laminin at a concentration of 10 µg/ml respectively. After differentiation medium for oligodendrocyte precursor cells was added to the 6-well plate, the neural stem cells prepared in Example 2 were seeded into the 6-well plate at a density of 4 x 10⁴ cells/cm².
3. After seeding, the differentiation medium for oligodendrocyte precursor cells was replaced every 48 hours. From the 8^{th} day of the seeding day when neural stem cells were seeded into the group 1 differentiation medium for oligodendrocyte precursor cells, oligodendrocyte precursor cells were digested with CTS Tryple Select and then collected. 2x10⁷ oligodendrocyte precursor cells could be obtained from 1 ml of bone marrow in Example 1 by the preparation method of oligodendrocyte precursor cells provided by the present invention. Therefore, the time for preparing oligodendrocyte precursor cells according to the differentiation medium and method provided by the present invention was significantly shortened compared with the prior art, and the production efficiency for oligodendrocyte precursor cells was improved. According to the cell counting results, the present invention has a higher yield of oligodendrocyte precursor cells.
4. Flow cytometry was used to detect the expression of Olig2, PDGFRα, NG2 and SOX10, wherein Olig2 is a marker specifically expressed by oligodendrocyte precursor cells. Figure 4 shows the rate of Olig2, PDGFRα, NG2 and SOX10 positive cells in the oligodendrocyte precursor cells prepared in the group 1 differentiation medium on the 10^{th} day, in which the positive expression rate of Olig2, PDGFRα, NG2 and SOX10 were 95.13%, 97.22%, 90.07% and 94.43%, respectively. For neural stem cells cultured in the group 2 differentiation medium on the 19^{th} day, the rate of Olig2, PDGFRα, NG2 and SOX10 positive cells were 72.5%, 69.36%, 75.91% and 74.75%, respectively. For neural stem cells cultured in the group 3 differentiation medium on the 17^{th} day, the rate of Olig2, PDGFRα, NG2 and SOX10 positive cells were 83.41%, 79.67%, 78.5% and 81.74%, respectively. The higher concentration of substances in the group 3 medium may transform oligodendrocyte precursor cells into oligodendrocytes, thereby reduce to some extent the rate of Olig2, PDGFRα, NG2 and SOX10 positive cells.

The addition of IGF-1 and Wnt3A to the differentiation medium can improve the differentiation efficiency of neural stem cells into oligodendrocyte precursor cells. For neural stem cells cultured in the group 4 differentiation medium on the 6^{th} day, the positive expression rate of Olig2, PDGFRα, NG2 and SOX10 were 79.11%, 84.75%, 84.32% and 87.69%, respectively. For neural stem cells cultured in the group 5 differentiation medium on the 6^{th} day, the positive expression rate of Olig2, PDGFRα, NG2 and SOX10 were 81.51%, 88.62%, 83.73% and 80.34%, respectively. For neural stem cells cultured in the group 6 differentiation medium on the 5^{th} day, the positive expression rate of Olig2, PDGFRα, NG2 and SOX10 were 95.35%, 98.81%, 95.33% and 94.17%, respectively.

For neural stem cells cultured in the group 7 differentiation medium on the 10^{th} day, the positive expression rates of Olig2, PDGFRα, NG2 and SOX10 were 67.73%, 71.95%, 69.25% and 66.09%, respectively, which were far lower than the purity of oligodendrocyte precursor cells cultured in the group 1 medium on the 10^{th}. In addition, the efficiency of preparing oligodendrocyte precursor cells in the differentiation medium without 2-mercaptoethanol was significantly reduced.

For neural stem cells cultured in the group 8 differentiation medium on the 10^{th} day, the positive expression rates of Olig2, PDGFRα, NG2 and SOX10 were 76.62%, 71.45%, 79.81% and 77.69%, respectively. For neural stem cells cultured in the group 9 differentiation medium on the 10^{th} day, the positive expression rates of Olig2, PDGFRα, NG2 and SOX10 positive cells were 71.37%, 68.45%, 72.69% and 71.11%, respectively. Through these two sets of experiments, it can be found that the medium containing both 2-mercaptoethanol and β-HRG1 has better effect on promoting the differentiation of neural stem cells into oligodendrocyte precursor cells than the medium containing both 2-mercaptoethanol and PDGF-AA.

For neural stem cells cultured in the group 10 differentiation medium on the 10^{th} day, the positive expression rates of Olig2, PDGFRα, NG2 and SOX10 were 72.44%, 74.96%, 75.31% and 71.33%, respectively. The results show that the combination of neurotrophic factor 3, 2-mercaptoethanol and PDGF-AA can also differentiate neural stem cells into oligodendrocyte precursor cells, but the differentiation efficiency is not as good as the combination of β-HRG1, 2-mercaptoethanol and PDGF-AA.

When the positive rates of the above four markers are all higher than 90%, the purity of the oligodendrocyte precursor cells in the cells is satisfied, which may be further used as an active ingredient of cell preparations or for clinical research.

### Example 5: In vitro experiments of oligodendrocyte precursor cells

1. Preparation of Myelin Forming Medium (MFM)
   CTS Neurobasal Medium containing 1% CTS GlutaMAX, 2% CTS B27, 10 µM 2-mercaptoethanol, and 1% P/S at a final concentration, as well as 10 µM T3 at a final concentration was prepared.
2. The oligodendrocyte precursor cells prepared from BMSC differentiation of Sprague-Dawley rat or human adult bone marrow by the above methods were co-cultured with neurons in MFM for 15 days, and the significantly increased expression of myelin basic protein was detected.

### Example 6: In vivo experiments of oligodendrocyte precursor cells

1. Preparation of Cell Injection Buffer (CIB)
   A Ca²⁺-free phenol solution or Mg²⁺-free Hank's Balanced Salt Solution (HBSS) was prepared, both containing 20 mM D-glucose and 1% P/S at a final concentration.
2.The oligodendrocyte precursor cells prepared in Example 4 were digested with CTS Tryple Select, CIB was added to terminate the digestion, centrifuged at 200 g for 5 min, and the precipitate was resuspend with CIB as an injection of oligodendrocyte precursor cells.
3. Animal experiments

The injection of oligodendrocyte precursor cells differentiated from human bone marrow and the injection of oligodendrocyte precursor cells differentiated from Sprague-Dawley rat bone marrow were injected into the central nervous system of shiverer model mice, respectively. The concentration of the injection was 4x10⁴ cells /ul. CIB without oligodendrocyte precursor cells was used as a control.

The shiverer model mouse is an experimental mouse that has myelin sheath damage in the central nervous system, which has a life span of only about 90 days and is widely used in the studies of myelin sheath damage.

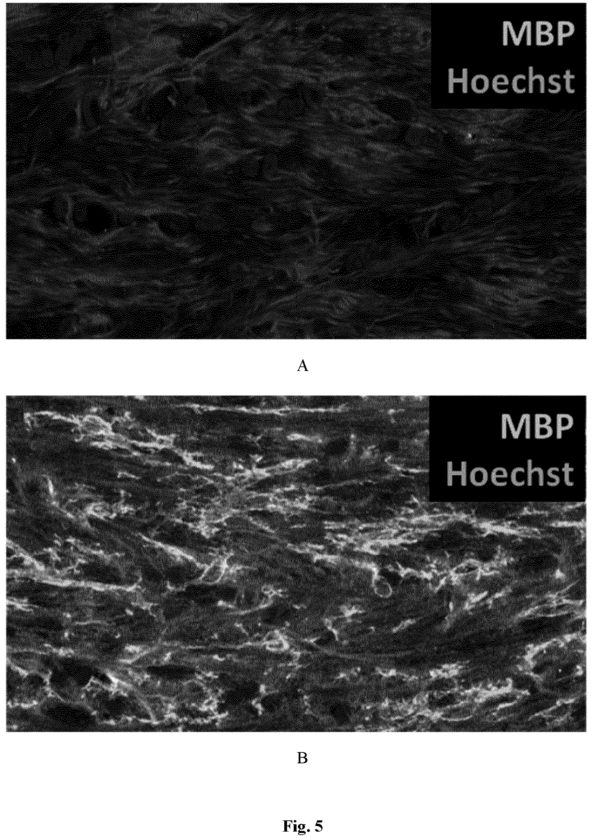

Figure 5 shows that 12 weeks from shiverer model mice were injected with oligodendrocyte precursor cells, the positive expression of a large amount of myelin basic protein (MBP) was detected in the brain tissue of the model mice, suggesting that oligodendrocyte precursor cells help the recovery of damaged myelin sheath, wherein the oligodendrocyte precursor cells were prepared by differentiation from rat bone marrow.

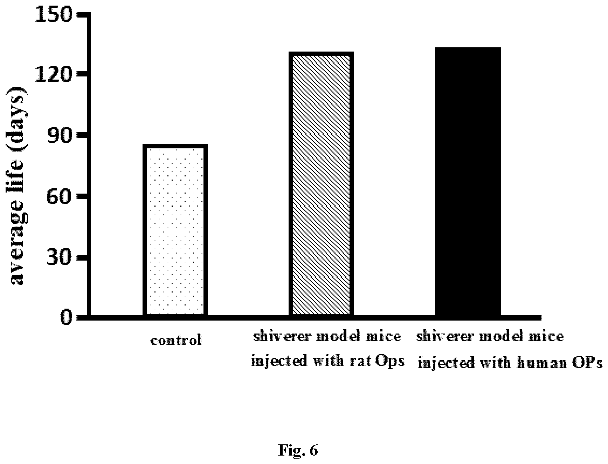

Figure 6 shows that oligodendrocyte precursor cells can extend the lifespan of shiverer model mice to an average of 125 days (up to 138 days). In addition, oligodendrocyte precursor cells prepared from human bone marrow have the same effect as oligodendrocyte precursor cells prepared from rat bone marrow, both can extend the life of model mice. Injection of oligodendrocyte precursor cells prepared from human bone marrow into shiverer model mice did not cause significant rejection.

The above experiment results show that the oligodendrocyte precursor cells differentiated from bone marrow mesenchymal stem cells using the differentiation conditions of the present invention have the potential to treat myelin sheath damage or other neurological diseases related to oligodendrocytes. The methods provided by the present invention significantly shorten the time for differentiation of bone marrow mesenchymal stem cells into oligodendrocyte precursor cells. Moreover, since the medium used in the present invention adopts a formula without animal-derived ingredients, the clinical application of oligodendrocyte precursor cells is further improved.

The immunofluorescence staining, flow cytometry and related reagents used in the cell identification and detection methods in the examples of the present invention are all conventional methods used by those skilled in the art for detecting or identifying corresponding cells.

The various experimental parameters disclosed in the examples of the present invention are experimental parameters screened by the inventors through multiple trials, which can effectively improve the differentiation efficiency of oligodendrocyte precursor cells. These do not limit the protection scope of the present invention. Obvious changes, substitutions or modifications to the culture medium compositions, contents or experimental conditions such as culturing time, centrifugal force, centrifugation time, culturing temperature on the basis of the present invention are all within the scope of the present invention.

## Claims

1. A medium for differentiating neural stem cells into oligodendrocyte precursor cells, wherein the medium comprises a basal medium mixed from Advanced DMEM/F12 and Neurobasal medium, as well as 0.5~2.5%GlutaMAX, 1~5% B27, 0.5~2.5%N2, 2~25 ng/ml bFGF, 2~25 ng/ml PDGF-AA, 25~250 ng/ml β-HRG1, 1~20 µM 2-mercaptoethanol and 1% P/S at a final concentration.

2. The medium according to claim 1, wherein the medium comprises a basal medium mixed from Advanced DMEM/F12 medium and Neurobasal medium at a volume ratio of 1:1, as well as 1% GlutaMAX, 2% B27, 1% N2, 10 ng/ml bFGF, 10 ng/ml PDGF-AA, 100 ng/ml β-HRG1, 10 µM 2-mercaptoethanol and 1% P/S at a final concentration.

3. The medium according to claim 1, wherein the medium comprises a basal medium mixed from Advanced DMEM/F12 and Neurobasal medium, as well as 0.5~2.5%GlutaMAX, 1~5% B27, 0.5~2.5%N2, 2~25 ng/ml bFGF, 2~25 ng/ml PDGF-AA, 2∼ 25 ng/mld IGF-1, 1-10 ng/ml Wnt3A, 25-250 ng/ml β-HRG1, 1-20 µM 2-mercaptoethanol and 1% P/S at a final concentration.

4. The medium according to claim 3, wherein the medium comprises a basal medium mixed from Advanced DMEM/F12 and Neurobasal medium at a volume ratio of 1:1, as well as 1% GlutaMAX, 2% B27, 1% N2, 10 ng/ml bFGF, 10 ng/ml PDGF-AA, 10 ng/ml IGF-1, 5 ng/ml ml Wnt3A, 100 ng/ml β-HRG1, 10 µM 2-mercaptoethanol and 1% P/S at a final concentration.

5. A method of preparing oligodendrocyte precursor cells using the medium according to any one of claims 1-4, comprising
preparing the medium of claim 1;
coating a cell culturing container with 2.5-25 µg/ml PLO and 2.5-25 µg/ml laminin, adding the medium of claim 1, seeding neural stem cells into the container at a density of 1 x 10⁴ to 5 × 10⁴ cells/cm²; and
replacing the medium of claim 1 regularly until oligodendrocyte precursor cells appear in the medium.

6. The method of preparing oligodendrocyte precursor cells according to claim 5, wherein the cell culture container is a 6-well plate.

7. The method of preparing oligodendrocyte precursor cells according to claim 5, wherein the neural stem cells are seeded into the container at a density of 4×10⁴ cells/cm².

8. The method of preparing oligodendrocyte precursor cells according to claim 5, wherein the neural stem cells are prepared by steps of:
preparing a medium for neural stem cells, wherein the medium is Advanced DMEM/F12 medium containing 0.5%∼2.5% GlutaMAX, 1%∼5% B27, 10~60 ng/ml bFGF, 10~60 ng/ml EGF, 5∼40 ng/ml IGF-1 and 1 % P/S at a final concentration;
culturing bone marrow mesenchymal stem cells by non-adherent culture using the medium for neural stem cells, and seeding the cells into a non-adherent cell culture container at a density of 7,500 to 20,000 cells/cm²; and
replacing the medium for neural stem cells regularly, wherein the neural stem cells appear in form of neurospheres in the medium.

9. The method of preparing oligodendrocyte precursor cells according to claim 8, wherein the neural stem cells are prepared by steps of:
preparing a medium for neural stem cells, wherein the medium is Advanced DMEM/F12 medium containing 1% GlutaMAX, 2% B27, 40 ng/ml bFGF, 40 ng/ml EGF, 20 ng/ml IGF-1 and 1 % P/S at a final concentration;
culturing bone marrow mesenchymal stem cells by non-adherent culture using the medium for neural stem cells, and
seeding the cells into a non-adherent cell culture container at a density of 10,000 cells/cm² for culturing.

10. The method of preparing oligodendrocyte precursor cells according to claim 8, wherein the neurospheres are isolated from the medium for neural stem cells by centrifuging at 100 to 300 g for 5 min.

11. The method of preparing oligodendrocyte precursor cells according to any one of claims 8 to 10, wherein the bone marrow mesenchymal stem cells are prepared from bone marrow by steps of:
culturing bone marrow in Stem Pro Medium, removing the medium and non-adherent cells 48h later, continuing culturing; and
after colonies of bone marrow mesenchymal stem cells appear in the culture, seeding the cells at a density of 40,000 cells/cm² into Stem Pro Medium for passage culturing, until the amount of CD45-positive cells in the medium is less than 1%.

12. The method of preparing oligodendrocyte precursor cells according to claim 11, wherein in the bone marrow mesenchymal stem cells used for preparing the neural stem cells, the amount of STRO-1, CD90 and CD73-positive cells is greater than 90%, the amount of Nestin-positive cells is greater than 5%, and the amount of CD45-positive cells is less than 1%.
